# EUROPÄISCHE PATENTSCHRIFT

(11) **EP 1 928 839 B1**
(45) Veröffentlichungstag und Bekanntmachung des Hinweises auf die Patenterteilung: **31.12.2008**
(21) Anmeldenummer: 06777164.2
(22) Anmeldetag: 05.09.2006
(51) Int. Cl.: C07D 231/16, B01J 31/02

(54) **VERFAHREN ZUM HERSTELLEN VON 5-FLUOR-1,3-DIALKYL-1H-PYRAZOL-4-CARBONSÄUREFLUORIDEN**
METHOD FOR THE PRODUCTION OF 5-FLUORO-1,3-DIALKYL-1H-PYRAZOL-4-CARBONYL FLUORIDES
PROCEDE POUR PRODUIRE DES FLUORURES DE L'ACIDE 5-FLUORO-1,3-DIALKYL-1H-PYRAZOL-4-CARBOXYLIQUE

(30) Priorität: 17.09.2005 DE 102005044451
(43) Veröffentlichungstag der Anmeldung: 11.06.2008
(73) Patentinhaber: Bayer CropScience AG, 40789 Monheim (DE)
(72) Erfinder: NEEFF, Arnd, 58256 Ennepetal (DE); PAZENOK, Sergiy, 42699 Solingen (DE)
(86) Internationale Anmeldenummer: PCT/EP2006/008635
(87) Internationale Veröffentlichungsnummer: WO 2007/031212

(56) Entgegenhaltungen:
- EP-A1- 0 776 889
- EP-A1- 1 266 904
- JP-A- 4 026 651
- A. PLESCHKE, A. MARHOLD, M. SCHNEIDER, A. KOLOMEITSEV, G.-V. RÖSCHENTHALER: "Halex reactions of aromatic compounds catalyzed by 2-azaallenium, carbophosphazenium, aminophosphonium and diphosphazenium salts: a comparative study" J. FLUORINE CHEM., Bd. 125, 2004, Seiten 1031-1038, XP004586028 in der Anmeldung erwähnt
- DATABASE WPI Week 1992 Derwent Publications Ltd., London, GB; AN 1992-084384 XP002418846 & JP 04 026651 A (ASAHI GLASS CO LTD) 29. Januar 1992 (1992-01-29)
- DATABASE WPI Week 198814 1988, Derwent Publications Ltd., London, GB; AN 1988-096363 XP002418742 & JP 63 048269 A (SUMITOMO) 29. Februar 1988 (1988-02-29)
- R. ERIC BANKS, KEVIN N. MOTHERSDALE, ANTHONY E. TIPPING, EFTHIMIOS TSILIOPOULOS: "Halex fluorination of chlorinated benzaldehydes and benzoyl chlorides" J. FLUORINE CHEM., Bd. 46, 1990, Seiten 529-537, XP002418739
- LANGLOIS B ET AL: "FLUORINATION OF AROMATIC COMPOUNDS BY HALOGEN EXCHANGE WITH FLUORIDE ANIONS (HALEX REACTION)" INDUSTRIAL CHEMISTRY LIBRARY, ELSEVIER, AMSTERDAM, NL, Bd. 8, 1996, Seiten 244-292, XP000865520 ISSN: 0926-9614

## Beschreibung

Die vorliegende Erfindung betrifft ein neues Verfahren zum Herstellen von 5-Fluor-1,3-dialkyl-1H-pyrazol-4-carbonsäurefluoriden, einer wertvollen Vorstufe für die Herstellung von Fungiziden, aus den entsprechenden 5-Chlor-1,3-dialkyl-1H-pyrazol-4-carbonsäurechloriden.

Es ist bereits bekannt, dass man 5-Fluor-1,3-dimethyl-1H-pyrazol-4-carbonsäurefluorid durch Umsetzung von 5-Chlor-1,3-dimethyl-1H-pyrazol-4-carbonsäurechlorid mit Kaliumfluorid in Gegenwart eines Verdünnungsmittels, bevorzugt Sulfolan, erhält (vgl. EP-A 0 776 889). Nachteilig ist an diesem Verfahren zum einen die geringe Raum/Zeit-Ausbeute, wodurch das Verfahren großtechnisch nur schwierig zu realisieren ist. Zum anderen lässt sich das Sulfolan nur schwierig durch Destillation vom Produkt abtrennen. In Anbetracht dieser Einschränkungen und Nachteile besteht Bedarf an einem verbesserten Verfahren für die Herstellung von 5-Fluor-1,3-dialkyl-1H-pyazol-4-carbonsäurefluoriden, durch das die den bekannten Verfahren innewohnenden Nachteile, insbesondere hohe Reaktionstemperaturen und lange Reaktionszeiten, vermieden und zudem die gewünschten Fluoride in guter bis sehr guter Ausbeute bei niedrigen Reaktionstemperaturen und kürzeren Reaktionszeiten erhalten werden.

Es ist bekannt, dass die Halex-Reaktion durch Zusatz von Phasentransfer-Katalysatoren (PTC) manchmal verbessert werden kann. Bislang wurden als Phasentransfer-Katalysatoren quartäre Alkylammonium-, Alkylphosphonium-, Pyridinium-, Amidophosphonium-, 2-Azaallenium-, Carbophosphazenium- und Diphosphazenium-Salze (EP-A 1 266 904) verwendet. Der direkte Vergleich von verschiedenen Katalysatoren zeigte, dass es keine generellen Regeln für das erfolgreiche Durchführen einer Halex-Reaktion gibt. Die Feineinstellungen betreffend Lösemittel, Lösung, Temperatur, Zusätze und Prozess müssen für jede Substanz individuell ausgeführt werden (vgl. J. Fluor. Chem. 2004, 125, 1031-1038).

Weiterhin ist bekannt, dass für den Erfolg der Fluorierung das Wasser weitgehend aus dem Reaktionsgemisch entfernt werden sollte. Für diese Zwecke benutzt man in der Produktion die azeotrope Trocknung in Gegenwart von Toluol oder Chlorbenzol. Das ist besonders wichtig bei der Fluorierung von Säurechloriden, um die Hydrolyse der Säurechlorid-Gruppe zu vermeiden. Die Fluorierung von 5-Chlor-1,3-dialkyl-1H-pyrazol-4-carbonsäurechloriden in Polyethylenglykoldimethylether (PEG) mit Siedepunkt von über 250°C könnte von großem Vorteil sein, da das Produkt direkt aus dem Reaktionsgemisch abdestilliert werden könnte. Kommerzielle PEG enthalten üblicherweise unterschiedliche Mengen Wasser (üblicherweise 0,6 bis 2 %). Um Wasser zu entfernen, wird das PEG azeotrop getrocknet, so dass der Gehalt an Wasser (Karl Fischer) unter 0,1 % sank. Das Lösemittel wurde für die Fluorierung von 5-Chlor-1,3-dialkyl-1H-pyrazol-4-carbonsäurechloriden eingesetzt (eigene Ergebnisse).

Wir haben festgestellt, dass die Fluorierung von 5-Chlor-1,3-dialkyl-1H-pyrazol-4-carbonsäurechloriden in PEG mit Wasser-Gehalt von unter 0,1 % mit Kaliumfluorid bei Temperaturen von 140°C bis 190°C binnen 12 Stunden keinen Erfolg brachte. Der Zusatz von herkömmlichen PTC wie quartäre Alkylammonium-, Alkylphosphonium-, Pyridinium-, Amidophosphonium-, 2-Azaallenium-, Carbophosphazenium- und Diphosphazenium-Salzen brachte nur geringfügige Verbesserung (Ausbeute 30 %).

Es wurde nun gefunden, dass man 5-Fluor-1,3-dialkyl-1H-pyrazol-4-carbonsäurefluoride der Formel (I) 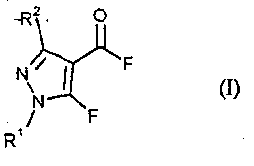 in welcher R¹ und R² unabhängig voneinander für C₁-C₃-Alkyl stehen, erhält, indem man

5-Chlor-1,3-dialkyl-1H-pyrazol-4-carbonsäurechloride der Formel (II) 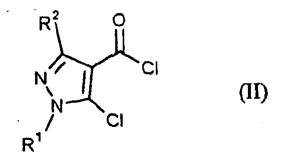 in welcher R¹ und R² die oben angegebenen Bedeutungen haben,
in der Gegenwart von Kaliumfluorid,
in Gegenwart eines Phasen-Transfer-Katalysators ausgewählt aus
(A) einer quartäre Phosphoniumverbindung der Formel (III) 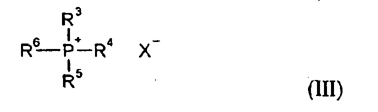 in welcher
   - R³, R⁴, R⁵ und R⁶: unabhängig voneinander für C₁-C₂₂-Alkyl, jeweils gegebenenfalls substitu- iertes Aryl oder (C₁-C₄-Alkyl)aryl steht, wobei Aryl die Bedeutung Phenyl oder Naphthyl hat und die besagten Substituenten Halogen, C₁-C₄-Alkyl, C₁-C₄-Alkoxy, Nitro oder Cyano bedeuten,
   - X⁻: für ein Anion steht,
   oder
(B) einem Amidophosphoniumsalz der Formel (IV) 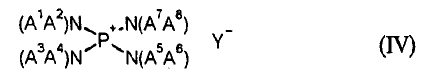 in welcher
   - A¹, A², A³, A⁴, A⁵, A⁶, A⁷ und A⁸: unabhängig voneinander für C₁-C₁₂-Alkyl oder C₂-C₁₂- Alkenyl, C₄-C₈-Cycloalkyl, C₆-C₁₂-Aryl, C₇-C₁₂-Aralkyl stehen, oder
   - A¹A², A³A⁴, A⁵A⁶ und A⁷A⁸: unabhängig voneinander direkt oder über O oder N-A⁹ miteinander zu einem 3- bis 7-gliedrigen Ring verbunden sind,
   - A⁹: für C₁-C₄-Alkyl steht,
   - Y⁻: einen einwertigen Säurerest oder das Äquivalent eines mehrwertigen Säurerests be- deutet,
   oder
(C) einer Verbindung der Formel (V) 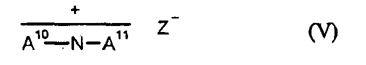 in welcher
   - A¹⁰ und A¹¹: unabhängig voneinander für einen der folgenden Reste stehen 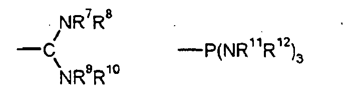
   - R⁷, R⁸, R⁹, R¹⁰, R¹¹ und R¹²: unabhängig voneinander für C₁-C₁₀-Alkyl, C₂-C₁₀-Alkenyl oder C₆-C₁₂-Aryl stehen, oder
   - R⁷R⁸, R⁹R¹⁰, R¹¹R¹²: unabhängig voneinander direkt miteinander zu einem 3- bis 5-gliedrigen, gesättigten oder ungesättigten Ring verbunden sind, der ein Stickstoffatom und ansonsten Kohlenstoffatome enthält wobei der Rest 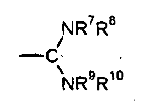 auch für einen gesättigten oder ungesättigten 4- bis 8-gliedrigen Ring stehen kann, der zwei Stickstoffatome und sonst Kohlenstoffatome enthält,

   - Z⁻: ein Äquivalent eines Anions bedeutet,
(D) einem Hexaalkylguanidiniumsalz der Formel (VI) 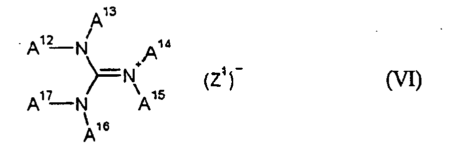 in welcher
   - A¹², A¹³, A¹⁴, A¹⁵, A¹⁶ und A¹⁷: unabhängig voneinander für C₁-C₁₂-Alkyl oder C₂-C₁₂-Alke- nyl, C₄-C₈-Cycloalkyl, C₆-C₁₂-Aryl, C₇-C₁₂-Aralkyl stehen, oder
   - A¹²A¹³, A¹⁴A¹⁵ und A¹⁶A¹⁷: unabhängig voneinander direkt oder über O oder N-A¹⁸ mitein- ander zu einem 3- bis 7-gliedrigen Ring verbunden sind,
   - A¹⁸: für C₁-C₄-Alkyl steht,
   - (Z¹)⁻: ein Äquivalent eines Anions bedeutet,
   in Gegenwart eines Polyethers der Formel (VII) 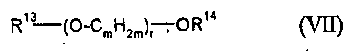 in welcher
   - R¹³ und R¹⁴: unabhängig voneinander für C₁-C₁₆-Alkyl stehen,
   - m⁻: für eine ganze Zahl von 2 bis 6 steht,
   - r: für eine ganze Zahl von 0 bis 20 steht,
und in Gegenwart von katalytischen Mengen einer protonhaltigen Verbindung aus der Gruppe: Sulfolan, Dimethylsulphoxid (DMSO), Dimethylacetamid, Dimethylformamid (DMF), N-Methylpyrrolidon (NMP), 1,3-Dimethylimidazolinon, HF, Wasser, Dichlormethan, Chloroform, Dichlorethan oder Trichlorethan; Ketone, wie Aceton, Butanon, Methyl-isobutylketon oder Cyclohexanon; Nitrile, wie Acetonitril, Propionitril, n- oder i-Butyronitril; Amide, wie N,N-Dimethylformamid, N,N-Dimethylacetamid, N-Methylformanilid, N-Methylpyrrolidon oder Hexamethylphosphorsäuretriamid, besonders bevorzugt Sulfolan, Dimethylsulphoxid, Dimethylacetamid , NMP oder Wasser, umsetzt.

Überraschenderweise lassen sich die 5-Fluor-1,3-dialkyl-1H-pyrazol-4-carbonsäurefluoride der Formel (I) unter den erfindungsgemäßen Bedingungen mit guten Ausbeuten in hoher Reinheit und Selektivität herstellen. Ein weiterer Vorteil des erfindungsgemäßen Verfahrens ist, dass die Reaktion schon bei 140-150°C stattfindet und dass das Produkt nach der Fluorierung direkt aus dem Reaktionsgemisch in reiner Form abdestilliert werden kann. Dabei fällt die Abtrennung des Produktes von Lösemittel komplett weg. Es wird vermutet, dass der Zusatz von katalytischen Mengen einer protonhaltigen Verbindung wie Sulfolan, DMF oder Wasser unter Reaktionsbedingungen in Gegenwart von KF (starke Base) kleine Mengen von HF bildet, welches die Reaktion (erste Stufe ist die Bildung von 5-Chlor-1,3-dialkyl-1H-pyrazol-4-carbonsäurefluorid) stark beschleunigt.

Verwendet man beispielsweise5-Chlor-1,3-dimethyl-1H-pyrazol-4-carbonsäurechlorid, Kaliumfluorid, Tetraphenylphosphoniumbromid, Polyethylenglycoldimethylether 500 und 0.1 Gew. % von Sulfolan als Ausgangsstoffe, so kann das erfindungsgemäße Verfahren im Detail durch das folgende Formelschema veranschaulicht werden:

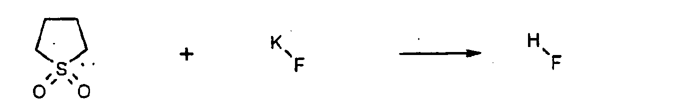

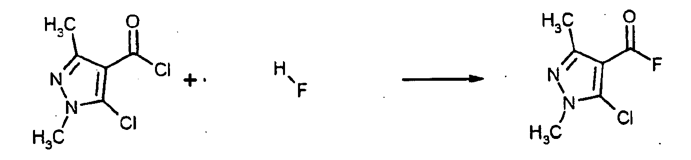

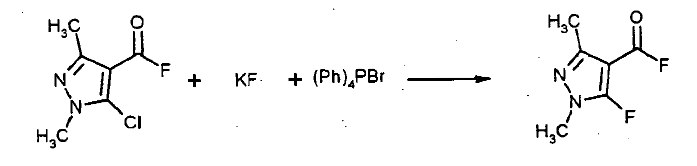

Die bei der Durchführung des erfindungsgemäßen Verfahrens als Ausgangsstoffe verwendeten 5-Chlor-1,3-dialkyl-1H-pyrazol-4-carbonsäurechloride sind durch die Formel (II) allgemein definiert. Die Reste R¹ und R² stehen in dieser Formel (II) unabhängig voneinander bevorzugt für Methyl, Ethyl, n-Propyl oder Isopropyl, besonders bevorzugt gleichzeitig für Methyl.

5-Chlor-1,3-dialkyl-1H-pyrazol-4-carbonsäurechloride der Formel (II) sind bekannt oder lassen sich nach bekannten Verfahren herstellen (vgl. EP-A 0 776 889).

Das weiterhin als Ausgangsstoff benötigte Kaliumfluorid ist eine bekannte Synthesechemikalie.

Die bei der Durchführung des erfindungsgemäßen Verfahrens als Phasen-Transfer-Katalysatoren verwendbaren quartären Phosphoniumverbindungen sind durch die Formel (III) allgemein definiert. R³, R⁴, R⁵ und R⁶ stehen in dieser Formel (III) unabhängig voneinander bevorzugt für C₁-C₁₈-Alkyl, für jeweils gegebenenfalls durch Fluor, Chlor, Brom, Methyl, Methoxy, Nitro oder Cyano substituiertes Phenyl oder Naphthyl, besonders bevorzugt für Butyl, Octyl, Hexadecyl, Octadecyl oder Phenyl. X- steht bevorzugt für ein Anion, wobei X aus der Reihe F, HF₂, Cl, I, Br, BF₄, ½ SO₄²⁻, Benzolsulfonyl, p-Toluolsulfonyl, HSO₄, PF₆ oder CF₃SO₃ ausgewählt ist. Ganz besonders bevorzugt sind die folgenden Phosphoniumverbindungen der Formel (III): Hexadecyltributylphosphoniumbromid, Stearyltributylphosphoniumbromid, Tetrabutylphosphoniumchlorid, Tetrabutylphosphoniumbromid, Tetraoctylphosphoniumbromid, Tetraphenylphosphoniumchlorid oder Tetraphenylphosphoniumbromid.
Phosphoniumverbindungen der Formel (III) sind bekannt (vgl. WO 98/32532).

Die bei der Durchführung des erfindungsgemäßen Verfahrens als Phasen-Transfer-Katalysatoren ebenfalls verwendbaren Amidophosphoniumsalze sind durch die Formel (IV) allgemein definiert. In dieser Formel (IV) stehen A¹, A², A³, A⁴, A⁵, A⁶, A⁷ und A⁸ unabhängig voneinander bevorzugt für C₁-C₈-Alkyl, C₂-C₈-Alkenyl, C₅-C₆-Cycloalkyl, besonders bevorzugt für C₁-C₄-Alkyl. Als Beispiele für Alkyl sind Methyl, Ethyl, n-Propyl, i-Propyl, n-Butyl, i-Butyl, n-Pentyl, 3-Methylbutyl, n-Hexyl, 2-Ethylhexyl, insbesondere Methyl, Ethyl, n-Propyl, n-Butyl und als Beispiele für Alkenyl sind Allyl, Prop-(2)-enyl, n-But-(2)-enyl, und als Beispiele für Cycloalkyl sind Cyclopentyl, Cyclohexyl, 4-Methylcyclohexyl, 4-tert.-Butylcyclohexyl, zu nennen. Es ist auch möglich, eine Verbindung der Formel (IV), worin A¹A² oder A¹A² und A³A⁴ oder A¹A² und A³A⁴ und A⁵A⁶ oder A¹A² und A³A⁴ und A⁵A⁶ und A⁷A⁸ direkt oder über O oder N-A⁹ miteinander zu einem gesättigten oder ungesättigten Ring mit 5 oder 6 Ringgliedern verbunden sind, einzusetzen. Dementsprechend enthalten diese Verbindungen einen, zwei, drei oder vier der vorstehend erwähnten Ringe. Es ist ferner möglich, eine Verbindung der Formel (IV), worin A¹A² oder A¹A² und A³A⁴ oder A¹A² und A³A⁴ und A⁵A⁶ oder A¹A² und A³A⁴ und A⁵A⁶ und A⁷A⁸ zu einem Ring, der das N-Atom, an dem die jeweiligen Reste A¹ bis A⁸ sitzen, gegebenenfalls O oder N-A⁹ und CH₂-Gruppen als Ringglieder umfasst, verbunden sind, einzusetzen. In dieser Stoffgruppe bilden das N-Atom mit den an ihnen jeweils befindlichen Resten A¹ bis A⁸ beispielsweise einen Hexahydropyridinring, Tetrahydropyrrolring, einen Hexahydropyrazinring oder Morpholinring. Dementsprechend enthalten diese Verbindungen einen, zwei, drei oder vier der vorstehend erwähnten Ringe. A⁹ steht bevorzugt für Methyl, Ethyl, n-Propyl, i-Propyl, n-Butyl, i-Butyl, sec-Butyl oder tert-Butyl.

In der Verbindung der Formel (IV) steht Y⁻ für einen einwertigen Säurerest oder das Äquivalent eines mehrwertigen Säurerestes, insbesondere den Rest einer anorganischen Mineralsäure, einer organischen Carbonsäure, einer aliphatischen oder aromatischen Sulfonsäure. Üblicherweise setzt man eine Verbindung der Formel (IV), worin Y für F, Cl, Br, S, HF₂, BF₄, Benzolsulfonyl, p-Toluolsulfonyl, HSO₄, PF₆, CF₃SO₃, insbesondere für F, Cl, Br, S, HF₂, BF₄, steht, ein. Als Beispiele für Verbindungen der Formel (IV) seien genannt: Tetrakis(dimethylamino)phosphoniumchlorid, Tetrakis-(diethylamino)phosphoniumchlorid, Tetrakis(dimethylamino)phosphoniumbromid, Tetrakis(diethylamino)phosphoniumbromid, Tetrakis(dipropylamino)phosphoniumchlorid oder -bromid, Tris(diethylamino)(dimethylamino)phosphoniumchlorid oder -bromid, Tetrakis(dibutylamino)phosphoniumchlorid oder -bromid, Tris(dimethylamino)(diethylamino)phosphoniumchlorid oder -bromid, Tris(dimethylamino)(cyclopentylamino)phosphoniumchlorid oder -bromid, Tris(dimethylamino)(dipropylamino)phosphoniumchlorid oder -bromid, Tris(dimethylamino)(dibutylamino)phosphoniumchlorid oder -bromid, Tris(dimethylamino)(cyclohexylamino)phosphoniumchlorid oder -bromid, Tris(dimethylamino)(diallylamino)phosphoniumchlorid oder -bromid, Tris(dimethylamino)(dihexylamino)phosphoniumchlorid oder -bromid, Tris(diethylamino)(dihexylamino)phosphoniumchlorid oder -bromid, Tris(dimethylamino)(diheptylamino)phosphoniumchlorid oder -bromid, Tris(diethylamino)(diheptylamino)phosphonium chlorid oder -bromid, Tetrakis(pyrrolidino)phosphoniumchlorid oder -bromid, Tetrakis(piperidino)phosphoniumchlorid oder -bromid, Tetrakis(morpholino)phosphoniumchlorid oder -bromid, Tris(piperidino)(diallyamino)phosphoniumchlorid oder -bromid, Tris-(pyrrolidino)(ethylmethylamino)phosphoniumchlorid oder -bromid, Tris(pyrrolidino)(diethylamino)-phosphoniumchlorid oder -bromid.

Amidophosphoniumsalze der Formel (IV) sind bekannt (vgl. WO 98/32532).

Die bei der Durchführung des erfindungsgemäßen Verfahrens als Phasen-Transfer-Katalysatoren ebenfalls verwendbaren Verbindungen sind durch die Formel (V) allgemein definiert. In dieser Formel (V) stehen R⁷, R⁸, R⁹, R¹⁰, R¹¹ und R¹² unabhängig voneinander bevorzugt für Methyl, Ethyl, n-Propyl, Isopropyl, n-, iso-, sec- oder tert-Butyl. R⁷ und R⁸, R⁹ und R¹⁰, R¹¹ und R¹² sind dabei bevorzugt identisch. Z⁻ steht in der Formel (V) für ein Äquivalent eines Anions, wobei Z ausgewählt ist aus Cl, Br, (CH₃)₃SiF₂, HF₂, H₂F₂, BF₄, PF₆, Carbonat oder Sulfat.

Besonders bevorzugte Verbindungen der Formel (V) sind durch die folgenden Formeln (V-1) bis (V-3) dargestellt:

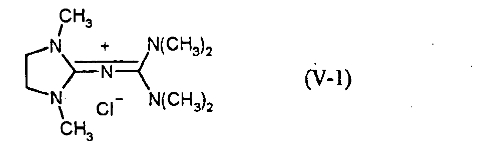

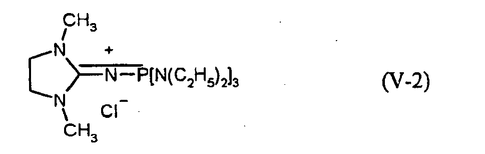

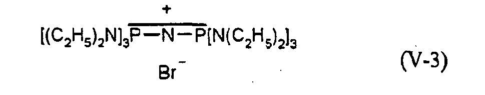

Die Verbindungen der Formel (V) sind bekannt (vgl. EP-A 1 266 904).

Die bei der Durchführung des erfindungsgemäßen Verfahrens als Phasen-Tränsfer-Katalysatoren ebenfalls verwendbaren Hexaalkylguanidiniumsalze sind durch die Formel (VI) allgemein definiert. In dieser Formel (VI) stehen A¹², A¹³, A¹⁴, A¹⁵, A¹⁶ und A¹⁷ unabhängig voneinander bevorzugt für C₁-C₈-Alkyl, C₂-C₈-Alkenyl, C₅-C₆-Cycloalkyl, besonders bevorzugt für C₁-C₄-Alkyl. Als Beispiele für Alkyl sind Methyl, Ethyl, n-Propyl, i-Propyl, n-Butyl, i-Butyl, n-Pentyl, 3-Methylbutyl, n-Hexyl, 2-Ethylhexyl, insbesondere Methyl, Ethyl, n-Propyl, n-Butyl und als Beispiele für Alkenyl sind Allyl, Prop-(2)-enyl, n-But-(2)-enyl, und als Beispiele für Cycloalkyl sind Cyclopentyl, Cyclohexyl, 4-Methylcyclohexyl, 4-tert.-Butylcyclohexyl, zu nennen. Es ist auch möglich, eine Verbindung der Formel (VI), worin A¹²A¹³ oder A¹²A¹³ und A¹⁴A¹⁵ oder A¹²A¹³ und A¹⁴A¹⁵ und A¹⁶A¹⁷ direkt oder über O oder N-A¹⁸ miteinander zu einem gesättigten oder ungesättigten Ring mit 5 oder 6 Ringgliedern verbunden sind, einzusetzen. Dementsprechend enthalten diese Verbindungen einen, zwei oder drei der vorstehend erwähnten Ringe. Es ist ferner möglich, eine Verbindung der Formel (VI), worin A¹²A¹³ oder A¹²A¹³ und A¹⁴A¹⁵ oder A¹²A¹³ und A¹⁴A¹⁵ und A¹⁶A¹⁷ zu einem Ring, der das N-Atom, an dem die jeweiligen Reste A¹ bis A⁸ sitzen, gegebenenfalls O oder N-A¹⁸ und CH₂-Gruppen als Ringglieder umfasst, verbunden sind, einzusetzen. In dieser Stoffgruppe bilden das N-Atom mit den an ihnen jeweils befindlichen Resten A¹² bis A¹⁷ beispielsweise einen Hexahydropyridinring, Tetrahydropyrrolring, einen Hexahydropyrazinring oder Morpholinring. Dementsprechend enthalten diese Verbindungen einen, zwei oder drei der vorstehend erwähnten Ringe. A¹⁸ steht bevorzugt für Methyl, Ethyl, n-Propyl, i-Propyl, n-Butyl, i-Butyl, sec-Butyl oder tert-Butyl. (Z¹)⁻ steht in der Formel (VI) für ein Äquivalent eines Anions, wobei Z¹ ausgewählt ist aus Cl, Br, (CH₃)₃SiF₂, HF₂, H₂F₂, BF₄, PF₆, Carbonat oder Sulfat.

Hexaalkyguanidiniumsalze der Formel (VI) sind bekannt (Synthesis, 1983, 904, US 5,082,968).

Die bei der Durchführung des erfindungsgemäßen Verfahrens weiterhin als Ausgangsstoffe benötigten Polyether sind durch die Formel (VII) allgemein definiert. In dieser Formel (VII) stehen R¹³ und R¹⁴ bevorzugt für C₁-C₈-Alkyl; m steht bevorzugt für 2 oder 3; r steht bevorzugt für eine ganze Zahl von 1 bis 18, insbesondere von 4 bis 14. Bevorzugte Polyether der Formel (VII) besitzen eine mittlere Molmasse zwischen 300 und 800 g/mol. Besonders bevorzugt ist ein Gemisch von Polyethylenglykoldimethylethern der Kettenlängen r von 6 bis 17 und einer mittleren Molmasse von 500 g/mol.

Polyether der Formel (VII) sind bekannt (vgl. WO 98/32532).

Erfindungsgemäß kann als Phasen-Transfer-Katalysator jede denkbare Kombination einer Verbindung der Formel (III) gemäß (A) mit einer Verbindung der Formel (IV) gemäß (B) oder mit einer Verbindung der Formel (V) gemäß (C) oder mit einem Gemisch aus (B) und (C), wobei die genannten Verbindungen gemäß (A), (B) und (C) selbst jeweils ebenfalls eine Mischung von entsprechenden Verbindungen sein können, eingesetzt werden. Ebenso sind alle möglichen Kombinationen mit einem Polyether der Formel (VII) denkbar.

Die Reaktionstemperaturen können bei der Durchführung des erfindungsgemäßen Verfahrens in einem größeren Bereich variiert werden. Im Allgemeinen arbeitet man bei Temperaturen von 120°C bis 150°C, bevorzugt bei Temperaturen von 130°C bis 150°C.

Bei der Durchführung des erfindungsgemäßen Verfahrens setzt man pro Mol an 5-Chlor-1,3-dialkyl-1H-pyrazol-4-carbonsäurechlorid der Formel (II) im Allgemeinen zwischen 0.5 und 3 mol %, bevorzugt 1-3 mol %, an Katalysator A, B, C oder D, und 2 bis 2.5 mol, bevorzugt 2.1 mol, von KF ein.

Die Menge von Polyethylenglykolether beträgt 0,25 bis 2 g, bevorzugt 0,5 bis 1 g pro Gramm von 5-Chlor-1,3-dialkyl-1H-pyrazol-4-carbonsäurechlorid der Formel (II). Die Menge von protonhaltigem Zusatz wie Sulfolan, NMP, Wasser etc. beträgt 0,01 bis 0,1 g pro Gramm von 5-Chlor-1,3-dialkyl-1H-pyrazol-4-carbonsäurechlorid der Formel (II).

Die Reaktionszeit kann in Abhängigkeit von der Reaktivität der Edukte bis zu 10 Stunden betragen, wobei der Abbruch der Reaktion bei vollständigem Umsatz auch schon früher erfolgen kann. Bevorzugt sind Reaktionszeiten von 3-5 Stunden.

Alle erfindungsgemäßen Verfahren werden im Allgemeinen unter Normaldruck durchgeführt. Es ist jedoch auch möglich, unter erhöhtem oder vermindertem Druck - im Allgemeinen zwischen 0,1 bar und 10 bar - zu arbeiten.

Die durch das erfindungsgemäßen Verfahren herstellbaren 5-Fluor-1,3-dialkyl-1H-pyrazol-4-carbonsäurefluoride der Formel (I) sind wertvolle Zwischenprodukte für die Herstellung von Fungiziden (vgl. z.B. EP-A 0 776 889).

Das erfindungsgemäße Herstellen von 5-Fluor-1,3-dialkyl-1H-pyrazol-4-carbonsäurefluoriden der Formel (I) wird in den nachstehenden Beispielen beschrieben, welche die obige Beschreibung weiter illustrieren. Die Beispiele sind jedoch nicht in einschränkender Weise zu interpretieren.

### Herstellungsbeispiele

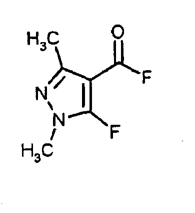

### Beispiel 1

Unter Argon werden 19.3 g (100 mmol) an 5-Chlor-1,3-dimethyl-1H-pyrazol-4-carbonsäurechlorid in Polyethylenglykoldimethylether (Wasser Gehalt KF 0.05 %) vorgelegt. Anschließend werden 17.43 g (300 mmol) Kaliumfluorid, 0,50 g (2 mmol) Bis(dimethylamino)[(1,3-dimethylimidazolidin-2-yliden)amino]methyliumchlorid und 100 mg Wasser zugegeben, auf 150°C erhitzt und bei dieser Temperatur für 5 Stunden nachgerührt. Anschließend wurde das Produkt unter Vakuum abdestilliert. Man erhält 13 g (82 % der Theorie) an 5-Fluor-1,3-dimethyl-1H-pyrazol-4-carbonsäurefluorid. S.p. 85-87 °C bei 10 mbar.

### Beispiel 2

Unter Argon werden 19.3 g (100 mmol) an 5-Chlor-1,3-dimethyl-1H-pyrazol-4-carbonsäurechlorid in Polyethylenglykoldimethylether (Wasser Gehalt KF 0.05 %) vorgelegt: Anschließend werden 17.43 g (300 mmol) Kaliumfluorid, 1 g Tetraphenylphosphoniumbromid und 300 mg Sulfolan zugegeben, auf 150°C erhitzt und bei dieser Temperatur für 5 Stunden nachgerührt. Anschließend wurde das Produkt unter Vakuum abdestilliert. Man erhält 13.3 g (83 % der Theorie) an 5-Fluor-1,3-dimethyl-1H-pyrazol-4-carbonsäurefluorid. Schmelzpunkt 85-87°C bei 10 mbar.

## Patentansprüche

1. Verfahren zum Herstellen von 5-Fluor-1,3-dialkyl-1H-pyrazol-4-carbonsäurefluoriden der Formel (I) 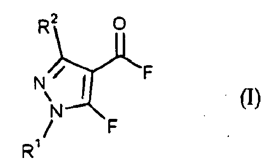 in welcher R¹ und R² unabhängig voneinander für C₁-C₃-Alkyl stehen,
**dadurch gekennzeichnet, dass** man
5-Chlor-1,3-dialkyl-1H-pyrazol-4-carbonsäurechloride der Formel (II) 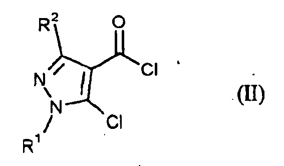 in welcher R¹ und R² die oben angegebenen Bedeutungen haben,
in der Gegenwart von Kaliumfluorid,
in Gegenwart eines Phasen-Transfer-Katalysators ausgewählt aus
(A) einer quartäre Phosphoniumverbindung der Formel (III) 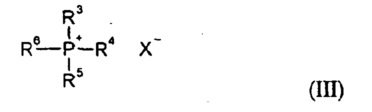 in welcher
R³, R⁴, R⁵ und R⁶ unabhängig voneinander für C₁-C₂₂-Alkyl, jeweils gegebenenfalls substituiertes Aryl oder (C₁-C₄-Alkyl)aryl steht, wobei Aryl die Bedeutung Phenyl oder Naphthyl hat und die besagten Substituenten Halogen, C₁-C₄- Alkyl, C₁-C₄-Alkoxy, Nitro oder Cyano bedeuten,
X⁻ für ein Anion steht,
oder
(B) einem Amidophosphoniumsalz der Formel (IV) 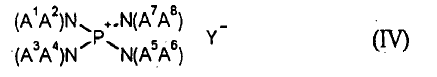 in welcher
A¹, A², A³, A⁴, A⁵, A⁶, A⁷ und A⁸ unabhängig voneinander für C₁-C₁₂-Alkyl oder C₂- C₁₂-Alkenyl, C₄-C₈-Cycloalkyl, C₆-C₁₂-Aryl, C₇-C₁₂-Aralkyl stehen, oder
A¹A², A³A⁴, A⁵A⁶ und A⁷A⁸ unabhängig voneinander direkt oder über O oder N-A⁹ miteinander zu einem 3- bis 7-gliedrigen Ring verbunden sind,
A⁹ für C₁-C₄-Alkyl steht,
Y⁻ einen einwertigen Säurerest oder das Äquivalent eines mehrwertigen Säurerests bedeutet,
oder
(C) einer Verbindung der Formel (V) 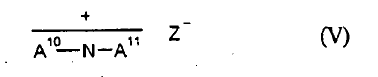 in welcher
A¹⁰ und A¹¹ unabhängig voneinander für einen der folgenden Reste stehen 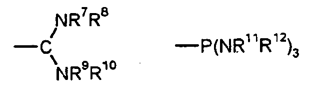
R⁷, R⁸, R⁹, R¹⁰, R¹¹ und R¹² unabhängig voneinander für C₁-C₁₀-Alkyl, C₂-C₁₀- Alkenyl oder C₆-C₁₂-Aryl stehen, oder
R⁷R⁸, R⁹R¹⁰, R¹¹R¹² unabhängig voneinander direkt miteinander zu einem 3- bis 5- gliedrigen, gesättigten oder ungesättigten Ring verbunden sind, der ein Stickstoffatom und ansonsten Kohlenstoffatome enthält
wobei der Rest 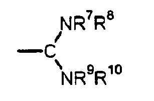 auch für einen gesättigten oder ungesättigten 4- bis 8-gliedrigen Ring stehen kann, der zwei Stickstoffatome und sonst Kohlenstoffatome enthält,
Z⁻ ein Äquivalent eines Anions bedeutet,
oder
(D) einem Hexaalkylguanidiniumsalz der Formel (VI) 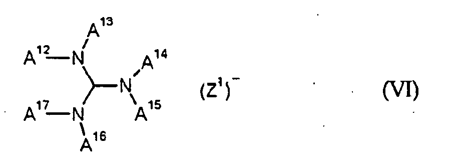 in welcher
A¹², A¹³, A¹⁴, A¹⁵, A¹⁶ und A¹⁷ unabhängig voneinander für C₁-C₁₂-Alkyl oder C₂- C₁₂-Alkenyl, C₄-C₈-Cycloalkyl, C₆-C₁₂-Aryl, C₇-C₁₂-Aralkyl stehen, oder
A¹²A¹³, A¹⁴A¹⁵ und A¹⁶A¹⁷ unabhängig voneinander direkt oder über O oder N-A¹⁸ miteinander zu einem 3- bis 7-gliedrigen Ring verbunden sind,
A¹⁸ für C₁-C₄-Alkyl steht,
(Z¹)⁻ ein Äquivalent eines Anions bedeutet,
in Gegenwart eines Polyethers der Formel (VII) 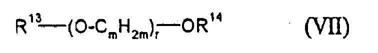 in welcher
R¹³ und R¹⁴ unabhängig voneinander für C₁-C₁₆-Alkyl stehen,
m für eine ganze Zahl von 2 bis 6 steht,
r für eine ganze Zahl von 0 bis 20 steht,
und in Gegenwart von katalytischen Mengen einer protonhaltigen Verbindung aus der Gruppe: Sulfolan, Dimethylsulphoxid (DMSO), Dimethylacetamid, Dimethylformamid (DMF), N-Methylpyrrolidon (NMP), 1,3-Dimethylimidazolinon, HF, Wasser, Dichlormethan, Chloroform, Dichlorethan oder Trichlorethan; Ketone, wie Aceton, Butanon, Methyl-isobutylketon oder Cyclohexanon; Nitrile, wie Acetonitril, Propionitril, n- oder i-Butyronitril; Amide, wie N,N-Dimethylformamid, N,N-Dimethylacetamid, N-Methylformanilid, N-Methylpyrrolidon oder Hexamethylphosphorsäuretriamid, besonders bevorzugt Sulfolan, Dimethylsulphoxid, Dimethylacetamid , NMP oder Wasser, umsetzt.

2. Verfahren gemäß Anspruch 1, **dadurch gekennzeichnet, dass** man 5-Chlor-1,3-dimethyl-1H-pyrazol-4-carbonsäurechlorid als Ausgangsstoff der Formel (II) einsetzt.

3. Verfahren gemäß Anspruch 1 oder 2, **dadurch gekennzeichnet, dass** man eine quartäre Phosphoniumverbindung der Formel (III) aus folgender Liste auswählt: Hexadecyltributylphosphoniumbromid, Stearyltributylphosphoniumbromid, Tetrabutylphosphoniumchlorid, Tetrabutylphosphoniumbromid, Tetraoctylphosphoniumbromid, Tetraphenylphosphoniumchlorid oder Tetraphenylphosphoniumbromid.

4. Verfahren gemäß Anspruch 1 oder 2, **dadurch gekennzeichnet, dass** ein Amidophosphoniumsalz der Formel (IV) aus folgender Liste auswählt: Tetrakis(dimethylamino)phosphoniumchlorid, Tetrakis(diethylamino)phosphoniumchlorid, Tetrakis(dimethylamino)phosphoniumbromid, Tetrakis(diethylamino)phosphoniumbromid, Tetrakis(dipropylamino)phosphoniumchlorid oder -bromid, Tris(diethylamino)(dimethylamino)phosphoniumchlorid oder -bromid, Tetrakis(dibutylamino)phosphoniumchlorid oder -bromid, Tris(dimethylamino)(diethylamino)phosphoniumchlorid oder -bromid, Tris(dimethylamino)(cyclopentylamino)phosphoniumchlorid oder -bromid, Tris(dimethylamino)(dipropylamino)phosphoniumchlorid oder -bromid, Tris(dimethylamino)(dibutylamino)phosphoniumchlorid oder -bromid, Tris(dimethylamino)(cyclohexylamino)phosphoniumchlorid oder -bromid, Tris(dimethylamino)(diallylamino)phosphoniumchlorid oder -bromid, Tris(dimethylamino)(dihexylamino)phosphoniumchlorid oder -bromid, Tris(diethylamino)(dihexylamino)phosphoniumchlorid oder -bromid, Tris(dimethylamino)(diheptylamino)phosphoniumchlorid oder -bromid, Tris(diethylamino)(diheptylamino)phosphonium chlorid oder -bromid, Tetrakis(pyrrolidino)phosphoniumchlorid oder -bromid, Tetrakis(piperidino)phosphoniumchlorid oder -bromid, Tetrakis(morpholino)phosphoniumchlorid oder -bromid, Tris(piperidino)(diallyiamino)phosphoniumchlorid oder -bromid, Tris(pyrrolidino)(ethylmethylamino)phosphoniumchlorid oder -bromid, Tris(pyrrolidino)(diethylamino)phosphoniumchlorid oder -bromid.

5. Verfahren gemäß Anspruch 1 oder 2, **dadurch gekennzeichnet, dass** man eine Verbindung der Formel (V) aus folgender Liste auswählt:

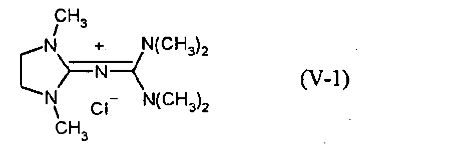

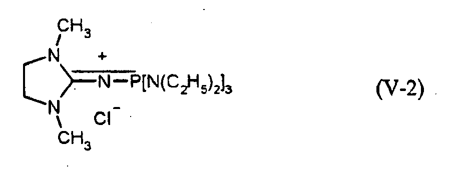

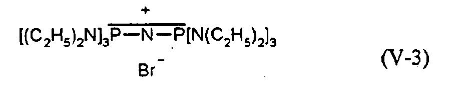

6. Verfahren gemäß Anspruch 1 oder 2, **dadurch gekennzeichnet, dass** man ein Hexaalkylguanidiniumsalz der Formel (VI) auswählt, worin A¹², A¹³, A¹⁴, A¹⁵, A¹⁶ und A¹⁷ unabhängig voneinander für C₁-C₈-Alkyl, C₂-C₈-Alkenyl, C₅-C₆-Cycloalkyl stehen; oder worin A¹²A¹³ oder A¹²A¹³ und A¹⁴A¹⁵ oder A¹²A¹³ und A¹⁴A¹⁵ und A¹⁶A¹⁷ direkt oder über O oder N-A¹⁸ miteinander zu einem gesättigten oder ungesättigten Ring mit 5 oder 6 Ringgliedern verbunden sind und A¹⁸ für Methyl, Ethyl, n-Propyl, i-Propyl, n-Butyl, i-Butyl, sec-Butyl oder tert-Butyl steht und (Z¹)⁻ ausgewählt ist aus Cl, Br, (CH₃)₃SiF₂, HF₂, H₂F₂, BF₄, PF₆, Carbonat oder Sulfat.

7. Verfahren gemäß Anspruch 1 oder 2, **dadurch gekennzeichnet, dass** man einen Polyether der Formel (VII) auswählt, worin R¹³ und R¹⁴ für C₁-C₈-Alkyl, m für 2 oder 3 und r für eine ganz Zahl von 1 bis 18 stehen.

8. Verfahren gemäß Anspruch 1 oder 2, **dadurch gekennzeichnet, dass** man einen Phasen-Transfer-Katalysator ausgewählt aus
(A) Tetraphenylphosphoniumbromid
oder
(C) Bis(dimethylamino)[(1,3-dimethylimidazolidin-2-yliden)amino]methyliumchlorid in Gegenwart von Polyethylenglykoldimethylether
und in Gegenwart von katalytischen Mengen Sulfolan umsetzt.

## Claims

1. Process for preparing 5-fluoro-1,3-dialkyl-1H-pyrazole-4-carbonyl fluorides of the formula (I) 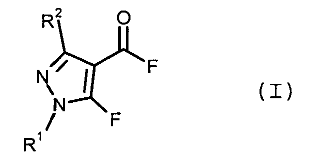 in which R¹ and R² are each independently C₁-C₃-alkyl,
**characterized in that**
5-chloro-1,3-dialkyl-1H-pyrazole-4-carbonyl chlorides of the formula (II) 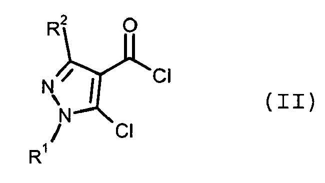 in which R¹ and R² are each as defined above
are converted in the presence of potassium fluoride,
in the presence of a phase transfer catalyst selected from
(A) a quaternary phosphonium compound of the formula (III) 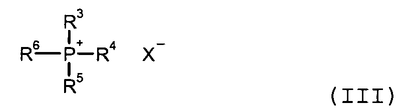 in which
R³, R⁴, R⁵ and R⁶ are each independently C₁-C₂₂- alkyl, in each case optionally substituted aryl or (C₁-C₄-alkyl)aryl, where aryl is defined as phenyl or naphthyl, and the said substituents are halogen, C₁-C₄-alkyl, C₁-C₄-alkoxy, nitro or cyano,
X⁻ is an anion,
or
(B) an amidophosphonium salt of the formula (IV) 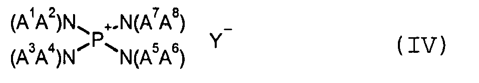 in which
A¹, A², A³, A⁴, A⁵, A⁶, A⁷ and A⁸ are each independently C₁-C₁₂-alkyl or C₂-C₁₂- alkenyl, C₄-C₈-cycloalkyl, C₆-C₁₂-aryl, C₇- C₁₂-aralkyl, or
A¹A², A³A⁴, A⁵A⁶ and A⁷A⁸ are each independently joined to one another directly or via O or N-A⁹ to give a 3- to 7-membered ring,
A⁹ is C₁-C₄-alkyl,
Y⁻ is a monobasic acid radical or the equivalent of a polybasic acid radical,
or
(C) a compound of the formula (V) 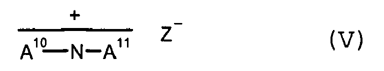 in which
A¹⁰ and A¹¹ are each independently one of the following radicals 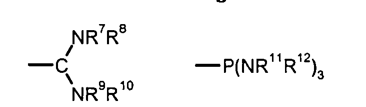
R⁷, R⁸, R⁹, R¹⁰, R¹¹ and R¹² are each independently C₁-C₁₀-alkyl, C₂-C₁₀-alkenyl or C₆-C₁₂-aryl, or
R⁷R⁸, R⁹R¹⁰, R¹¹R¹² are each independently joined directly to one another to give a 3- to 5-membered, saturated or unsaturated ring which contains one nitrogen atom and otherwise carbon atoms,
where the radical 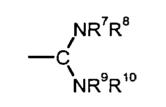 may also be a saturated or unsaturated, 4- to atoms 8-membered ring which contains two nitrogen and otherwise carbon atoms,
Z⁻ is an equivalent of an anion,
or
(D) a hexaalkylguanidinium salt of the formula (VI) 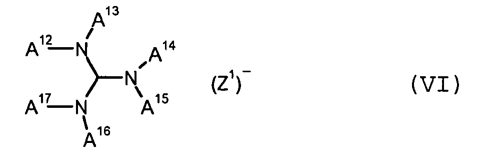 in which
A¹², A¹³, A¹⁴, A¹⁵, A¹⁶ and A¹⁷ are each independently C₁-C₁₂-alkyl or C₂-C₁₂-alke- nyl, C₄-C₈-cycloalkyl, C₆-C₁₂-aryl, C₇-C₁₂- aralkyl, or
A¹²A¹³, A¹⁴A¹⁵ and A¹⁶A¹⁷ are each independently joined to one another directly or via O or N-A¹⁸ to give a 3- to 7-membered ring,
A¹⁸ is C₁-C₄-alkyl,
(Z¹)⁻ is one equivalent of an anion,
in the presence of a polyether of the formula (VII) 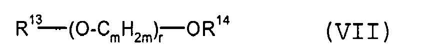 in which
R¹³ and R¹⁴ are each independently C₁-C₁₆-alkyl,
m is an integer from 2 to 6,
r is an integer from 0 to 20,
and in the presence of catalytic amounts of a protic compound from the group of sulpholane, dimethyl sulphoxide (DMSO), dimethylacetamide, dimethylformamide (DMF), N-methylpyrrolidone (NMP), 1,3-dimethylimidazolinone, HF, water, dichloromethane, chloroform, dichloroethane or trichloroethane; ketones such as acetone, butanone, methyl isobutyl ketone or cyclohexanone; nitriles such as acetonitrile, propionitrile, nor i-butyronitrile; amides such as N,N-dimethylformamide, N,N-dimethylacetamide, N-methylformanilide, N-methylpyrrolidone or hexamethylphosphoramide, more preferably sulpholane, dimethyl sulphoxide, dimethylacetamide, NMP or water.

2. Process according to Claim 1, **characterized in that** 5-chloro-1,3-dimethyl-1H-pyrazole-4-carbonyl chloride is used as the starting material of the formula (II).

3. Process according to Claim 1 or 2, **characterized in that** a quaternary phosphonium compound of the formula (III) is selected from the following list: hexadecyltributylphosphonium bromide, stearyltributylphosphonium bromide, tetrabutylphosphonium chloride, tetrabutylphosphonium bromide, tetraoctylphosphonium bromide, tetraphenylphosphonium chloride or tetraphenylphosphonium bromide.

4. Process according to Claim 1 or 2, **characterized in that** an amidophosphonium salt of the formula (IV) is selected from the following list: tetrakis(dimethylamino)phosphonium chloride, tetrakis(diethylamino)phosphonium chloride, tetrakis(dimethylamino)phosphonium bromide, tetrakis(diethylamino)phosphonium bromide, tetrakis(dipropylamino)phosphonium chloride or bromide, tris(diethylamino)(dimethylamino)phosphonium chloride or bromide, tetrakis(dibutylamino)phosphonium chloride or bromide, tris(dimethylamino)(diethylamino)phosphonium chloride or bromide, tris(dimethylamino)(cyclopentylamino)phosphonium chloride or bromide, tris(dimethylamino)(dipropylamino)phosphonium chloride or bromide, tris(dimethylamino)(dibutylamino)phosphonium chloride or bromide, tris(dimethylamino)(cyclohexylamino)phosphonium chloride or bromide, tris(dimethylamino)(diallylamino)phosphonium chloride or bromide, tris(dimethylamino)(dihexylamino)phosphonium chloride or bromide, tris(diethylamino)(dihexylamino)phosphonium chloride or bromide, tris-(dimethylamino)(diheptylamino)phosphonium chloride or bromide, tris(diethylamino)-(diheptylamino)phosphonium chloride or bromide, tetrakis(pyrrolidino)phosphonium chloride or bromide, tetrakis(piperidino)phosphonium chloride or bromide, tetrakis(morpholino)phosphonium chloride or bromide, tris(piperidino)(diallylamino)phosphonium chloride or bromide, tris(pyrrolidino)(ethylmethylamino)phosphonium chloride or bromide, tris(pyrrolidino)(diethylamino)phosphonium chloride or bromide.

5. Process according to Claim 1 or 2, **characterized in that** a compound of the formula (V) is selected from the following list:

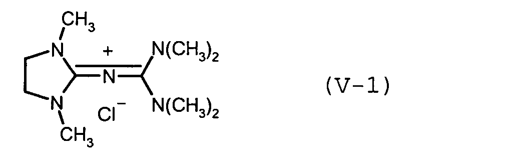

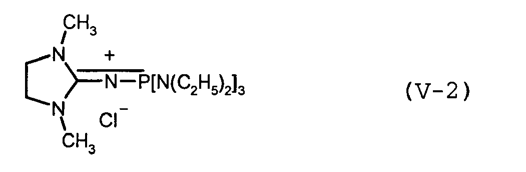

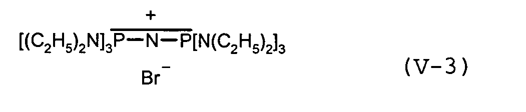

6. Process according to Claim 1 or 2, **characterized in that** a hexaalkylguanidinium salt of the formula (VI) in which A¹², A¹³, A¹⁴, A¹⁵, A¹⁶ and A¹⁷ are each independently C₁-C₈-alkyl, C₂-C₈-alkenyl, C₅-C₆-cycloalkyl; or in which A¹²A¹³, or A¹²A¹³ and A¹⁴A¹⁵, or A¹²A¹³ and A¹⁴A¹⁵ and A¹⁶A¹⁷, are joined to one another directly or via O or N-A¹⁸ to give a saturated or unsaturated ring having 5 or 6 ring members, and A¹⁸ is methyl, ethyl, n-propyl, i-propyl, n-butyl, i-butyl, sec-butyl or tert-butyl, and (Z¹)⁻ is selected from Cl, Br, (CH₃)₃SiF₂, HF₂, H₂F₂, BF₄, PF₆, carbonate or sulphate is selected.

7. Process according to Claim 1 or 2, **characterized in that** a polyether of the formula (VII) in which R¹³ and R¹⁴ are each C₁-C₈-alkyl, m is 2 or 3 and r is an integer from 1 to 18 is selected.

8. Process according to Claim 1 or 2, **characterized in that** a phase transfer catalyst selected from
(A) tetraphenylphosphonium bromide
or
(C) bis(dimethylamino)[(1,3-dimethylimidazolidin-2-ylidene)amino]methylium chloride is converted in the presence of polyethylene glycol dimethyl ether
and in the presence of catalytic amounts of sulpholane.

## Revendications

1. Procédé de production de fluorures d'acides 5-fluoro-1,3-dialkyl-1H-pyrazole-4-carboxyliques de formule (I) 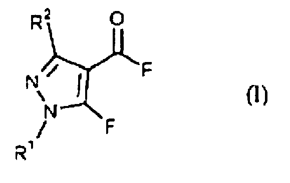 dans laquelle R¹ et R² représentent, indépendamment l'un de l'autre, un reste alkyle en C₁ à C₃,
**caractérisé en ce qu'**on fait réagir
des chlorures d'acides 5-chloro-1,3-dialkyl-1H-pyrazole-4-carboxyliques de formule (II) 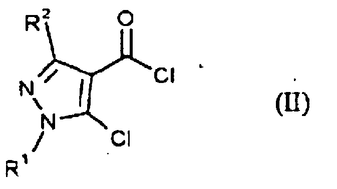 dans laquelle R¹ et R² ont les définitions indiquées ci-dessus,
en présence de fluorure de potassium,
en présence d'un catalyseur de transfert de phase choisi entre
(A) un composé de phosphonium quaternaire de formule (III) 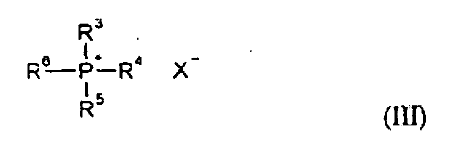 dans laquelle
R³, R⁴, R⁵ et R⁶ représentent, indépendamment les uns des autres, un reste alkyle en C₁ à C₂₂, aryle ou (alkyle en C₁ à C₄)aryle, chacun éventuellement substitué, aryle étant défini comme représentant un reste phényle ou naphtyle et les substituants en question représentant un halogène, un reste alkyle en C₁ à C₄, alkoxy en C₁ à C₄, un groupe nitro ou cyano,
X⁻ représente un anion,
ou bien
(B) un reste d'amidophosphonium de formule (IV) 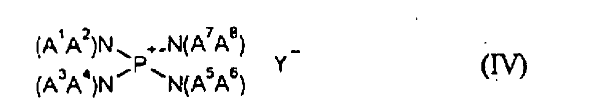 dans laquelle
A¹, A², A³, A⁴, A⁵, A⁶, A⁷ et A⁸ représentent, indépendamment les uns des autres, un reste alkyle en C₁ à C₁₂ ou un reste alcényle en C₂ à C₁₂, cycloalkyle en C₄ à C₈, aryle en C₆ à C₁₂, aralkyle en C₇ à C₁₂, ou bien
A¹A², A³A⁴, A⁵A⁶ et A⁷A⁸, indépendamment les uns des autres, liés ensemble en un noyau trigonal à heptagonal, directement ou par l'intermédiaire de O ou de N-A⁹,
A⁹ représente un reste alkyle en C₁ à C₄,
Y⁻ représente un reste acide monovalent ou l'équivalent d'un reste acide polyvalent,
ou bien
(C) un composé de formule (V) 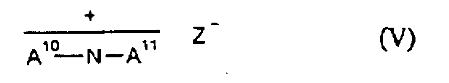 dans laquelle
A¹⁰ et A¹¹ représentent, indépendamment l'un de l'autre, l'un des restes suivants 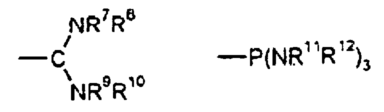
R⁷, R⁸, R⁹, R¹⁰, R¹¹ et R¹² représentent indépendamment les uns des autres un reste alkyle en C₁ à C₁₀, alcényle en C₂ à C₁₀ ou aryle en C₆ à C₁₂, ou bien
R⁷R⁸, R⁹R¹⁰, R¹¹R¹², indépendamment les uns des autres, sont liés directement ensemble en un noyau trigonal à pentagonal, saturé ou non saturé, qui contient un atome d'azote, les autres atomes étant des atomes de carbone, le reste 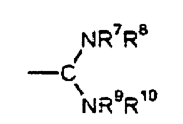 pouvant aussi représenter un noyau tétragonal à heptagonal saturé ou non saturé, qui contient deux atomes d'azote, les autres atomes étant des atomes de carbone,
Z⁻ désigne un équivalent d'un anion,
ou bien
(D) un sel d'hexaalkylguanidinium de formule (VI) 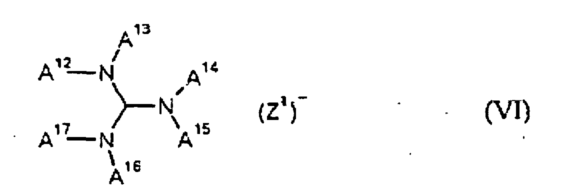 dans laquelle
A¹², A¹³, A¹⁴, A¹⁵, A¹⁶ et A¹⁷ représentent, indépen- damment les uns des autres, un reste alkyle en C₁ à C₁₂ ou un reste alcényle en C₂ à C₁₂, cycloalkyle en C₄ à C₈, aryle en C₆ à C₁₂, aralkyle en C₇ à C₁₂, ou bien
A¹²A¹³, A¹⁴A¹⁵ et A¹⁶A¹⁷, indépendamment les uns des autres, sont liés ensemble en un noyau trigonal à heptagonal directement ou par l'intermédiaire de O ou de N-A¹⁸,
A¹⁸ représente un reste alkyle en C₁ à C₄,
(Z¹)⁻ représente un équivalent d'un anion,
en présence d'un polyéther de formule (VII) 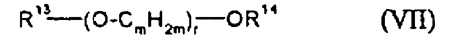 dans laquelle
R¹³ et R¹⁴ représentent, indépendamment l'un de l'autre, un reste alkyle en C₁ à C₁₆,
m est un nombre entier de 2 à 6,
r est un nombre entier de 0 à 20,
et en présence de quantités catalytiques d'un composé protoné du groupe :
sulfolan, diméthylsulfoxyde (DMSO), diméthylacétamide, diméthylformamide (DMF), N-méthylpyrrolidone (NMP), 1,3-diméthylimidazolinone, HF, eau, dichlorométhane, chloroforme, dichloréthane ou trichloréthane ; de cétones telles qu'acétone, butanone, méthylisobutylcétone ou cyclohexanone ; de nitriles, tels qu'acétonitrile, propionitrile, n-butyronitrile ou isobutyronitrile ; d'amides telles que N,N-diméthylformamide, N,N-diméthylacétamide, N-méthylformanilide, N-méthylpyrrolidone ou triamide d'acide hexaméthylphosphorique, très avantageusement sulfolane, diméthylsulfoxyde, diméthylacétamide, NMP ou eau.

2. Procédé selon la revendication 1, **caractérisé en ce qu'**on utilise le chlorure d'acide 5-chloro-1,3-diméthyl-1H-pyrazole-4-carboxylique comme composé de départ de formule (II).

3. Procédé suivant la revendication 1 ou 2, **caractérisé en ce qu'**on choisit un composé de phosphonium quaternaire de formule (III) dans la liste suivante : bromure d'hexadécyltributylphosphonium, bromure de stéaryltributylphosphonium, chlorure de tétrabutylphosphonium, bromure de tétrabutylphosphonium, bromure de tétraoctylphosphonium, chlorure de tétraphénylphosphonium ou bromure de tétraphénylphosphonium.

4. Procédé suivant la revendication 1 ou 2, **caractérisé en ce qu'**on choisit un sel d'amidophosphonium de formule (IV) dans la liste suivante : chlorure de tétrakis(diméthylamino)phosphonium, chlorure de tétrakis-(diéthylamino)phosphonium, bromure de tétrakis(diméthylamino)phosphonium, bromure de tétrakis(diéthylamino)-phosphonium, chlorure ou bromure de tétrakis(dipropylamino)phosphonium, chlorure ou bromure de tris(diéthylamino)(diméthylamino)phosphonium, chlorure ou bromure de tétrakis(dibutylamino)phosphonium, chlorure ou bromure de tris(diméthylamino)(diéthylamino)phosphonium, chlorure ou bromure de tris(diméthylamino)(cyclopentylamino)phosphonium, chlorure ou bromure de tris-(diméthylamino)-(dipropylamino)phosphonium, chlorure ou bromure de tris(diméthylamino)(dibutylamino)phosphonium, chlorure ou bromure de tris(diméthylamino)(cyclohexylamino)phosphonium, chlorure ou bromure de tris(diméthylamino)(diallylamino)phosphonium, chlorure ou bromure de tris(diméthylamino)(dihexylamino)phosphonium, chlorure ou bromure de tris(diéthylamino)(dihexylamino)phosphonium, chlorure ou bromure de tris(diméthylamino)(diheptylamino)phosphonium, chlorure ou bromure de tris(diéthylamino)(diheptylamino)phosphonium, chlorure ou bromure de tétrakis(pyrrolidino)phosphonium, chlorure ou bromure de tétrakis-(pipéridino)phosphonium, chlorure ou bromure de tétrakis(morpholino)phosphonium, chlorure ou bromure de tris(pipéridino)(diallylamino)phosphonium, chlorure ou bromure de tris(pyrrolidino)(éthylméthyl-amino)phosphonium, chlorure ou bromure de tris(pyrrolidino)(diéthylamino)phosphonium.

5. Procédé suivant la revendication 1 ou 2, **caractérisé en ce qu'**on choisit un composé de formule (V) dans la liste suivante :

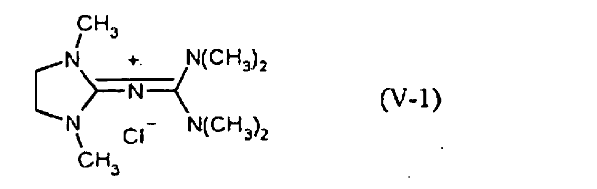

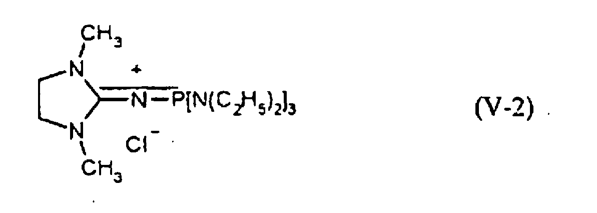

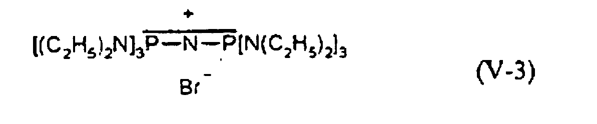

6. Procédé suivant la revendication 1 ou 2, **caractérisé en ce qu'**on choisit un sel d'hexaalkylguanidinium de formule (VI) dans laquelle A¹², A¹³, A¹⁴, A¹⁵, A¹⁶ et A¹⁷ représentent, indépendamment les uns des autres, un reste alkyle en C₁ à C₈, alcényle en C₂ à C₈, cycloalkyle en C₅ ou C₆ ; ou dans laquelle A¹²A¹³ ou A¹²A¹³ et A¹⁴A¹⁵ ou A¹²A¹³ et A¹⁴A¹⁵ et A¹⁶A¹⁷ sont liés ensemble directement ou par l'intermédiaire de O ou de N-A¹⁸ en un noyau pentagonal ou hexagonal saturé ou non saturé et A¹⁸ représente un reste méthyle, éthyle, n-propyle, isopropyle, n-butyle, isobutyle, sec.-butyle ou tertiobutyle et (Z¹)⁻ est choisi entre Cl, Br, (CH₃)₃SiF₂, HF₂, H₂F₂, BF₄, PF₆, carbonate ou sulfate.

7. Procédé suivant la revendication 1 ou 2, **caractérisé en ce qu'**on choisit un polyéther de formule (VII) dans laquelle R¹³ et R¹⁴ représentent un reste alkyle en C₁ à C₈, m a la valeur de 2 ou 3 et r représente un nombre entier de 1 à 18.

8. Procédé suivant la revendication 1 ou 2, **caractérisé en ce qu'**on choisit un catalyseur de transfert de phases entre
(A) le bromure de tétraphénylphosphonium ou
(C) le chlorure de bis(diméthylamino)[(1,3-diméthylimidazolidine-2-ylidène)amino]méthylium en présence d'éther diméthylique de polyéthylèneglycol
et on conduit la réaction en présence de quantités catalytiques de Sulfolane.
